(19)

Europäisches
Patentamt
European
Patent Office
Office européen
des brevets

(11) **EP 4 417 196 A1**

(12) **EUROPEAN PATENT APPLICATION**

(43) Date of publication:
**21.08.2024 Bulletin 2024/34**

(21) Application number: **24157768.3**

(22) Date of filing: **15.02.2024**

(51) International Patent Classification (IPC):
**A61K 9/28** *(2006.01)*

(52) Cooperative Patent Classification (CPC):
**A61K 9/2866; A61K 9/2893**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.02.2023 JP 2023023059**

(71) Applicant: **Shin-Etsu Chemical Co., Ltd.
Tokyo 100-0005 (JP)**

(72) Inventors:
• **FURUYA, Shun
 Joetsu-shi, 942-8601 (JP)**
• **HIRAMA, Yasuyuki
 Joetsu-shi, 942-8601 (JP)**

(74) Representative: **Cabinet Nony
11 rue Saint-Georges
75009 Paris (FR)**

(54) **AQUEOUS COMPOSITION COMPRISING HYDROXYPROPYL METHYL CELLULOSE ACETATE SUCCINATE AND METHOD FOR PRODUCING SOLID PREPARATION**

(57)    Provided is an aqueous composition that is acid-resistant, does not cause blockage of spray nozzle even without a cooling equipment, and can be suitably used in an enteric coating preparation in a simple and convenient manner. The aqueous composition includes hydroxypropyl methyl cellulose acetate succinate having an acetyl group substitution degree from 0.10 to 2.50 and a succinyl group substitution degree from 0.05 to 2.50 per number of anhydroglucose unit; a neutralizer; and water. The degree of neutralization of the hydroxypropyl methyl cellulose acetate succinate by the neutralizer is from 130 to 260 mol %.

**EP 4 417 196 A1**

**Description**

[Technical field]

The present invention relates to an aqueous composition comprising hydroxypropyl methyl cellulose acetate succinate (also referred to as "HPMCAS" hereunder) and applications thereof.

[Background art]

Some drugs used in pharmaceutical applications are unstable with respect to acids or cause irritation or injury to the gastric mucosa. An enteric coating is applied to protect such drugs from gastric acidity and to protect the gastric mucosa from irritation and damage caused by drugs.

Conventionally, the enteric coating has been performed by dissolving an enteric polymer in an organic solvent. However, a large amount of organic solvent used are released into the atmosphere, causing many problems including environmental protection and safety concerns as well as toxicity due to residual organic solvents in pharmaceuticals.

In order to circumvent these problems, aqueous enteric coatings have been developed. Some methods have been known that include a method of dispersing a micronized enteric polymer in water containing a plasticizer such as triacetin under temperature control to make a coating liquid (Patent document 1) and a method of partially neutralizing the enteric polymer with an aqueous ammonia solution (Patent document 2).

[Prior art documents]

[Patent documents]

[Patent document 1] JP-A-55-98120
[Patent document 2] JP-T-2018-532013

[Summary of the invention]

[Problems to be solved by the invention]

However, the invention described in JP-A-55-98120 requires cooling of the path from the tank where the coating liquid is stored to the spray nozzle in order to prevent nozzle blockage caused by adhesion and aggregation of the enteric polymer during the coating process.

In the invention described in JP-T-2018-532013, it is necessary to prepare an aqueous solution by dissolving a cold-water soluble enteric polymer with a low total ester substitution degree in cold water, and further, it is necessary to mix the aqueous solution and an aqueous dispersion liquid of the enteric polymer. Consequently, there has been a demand for the development of a more convenient method of producing an enteric coating preparation.

The present invention was made to solve the aforementioned problems, and it is an object of the present invention to provide an aqueous composition suitable for an enteric coating preparation having acid resistance equivalent or superior to that of conventional aqueous enteric coating preparations by a simple method without requiring a cooling equipment.

[Means to solve the problems]

The inventors of the present invention diligently conducted a series of studies to achieve the above object, and completed the invention as follows. That is, the inventors found that the viscosity of the aqueous composition can be reduced by adding an excess amount of a neutralizer to an aqueous dispersion liquid of HPMCAS, that the aqueous composition can be prepared even at room temperature, and that an enteric-coated preparation can be easily and efficiently produced by increasing the concentration of HPMCAS.

The present invention is to provide the following aqueous compositions and a method for producing a solid preparation(s).

[1] An aqueous composition including hydroxypropyl methyl cellulose acetate succinate having an acetyl group substitution degree from 0.10 to 2.50 and a succinyl group substitution degree from 0.05 to 2.50 per number of anhydroglucose unit, a neutralizer, and water, wherein the degree of neutralization of the hydroxypropyl methyl cellulose acetate succinate by the neutralizer is from 130 to 260 mol %.

[2] The aqueous composition according to [1], wherein the neutralizer is at least one selected from the group consisting of ammonia and alkylamine.

[3] The aqueous composition according to [1] or [2], wherein the aqueous composition is for enteric coating.

[4] A method for producing a solid preparation including: applying the aqueous composition according to [3] to a core containing at least an active ingredient, and drying the same thereafter.

[Effects of the invention]

According to the present invention, an aqueous composition with excellent acid resistance can be provided without requiring special HPMCAS. Further, since the composition of the present invention is aqueous, it is not necessary to use an organic solvent, and it requires no special equipment such as a cooling equipment for its production, and thus can be produced in a simple manner. Moreover, the low viscosity of the composition allows the concentration of HPMCAS to be increased, thereby reducing production costs by shortening the coating time.

[Mode for carrying out the invention]

1. Aqueous composition

First, hereunder described is an aqueous composition containing HPMCAS with an acetyl group substitution from 0.10 to 2.50 per number of anhydroglucose unit and a succinyl group substitution from 0.05 to 2.50 per number of anhydroglucose unit, a neutralizer, and water.

HPMCAS is a cellulose derivative having four kinds of substituent groups in cellulose: a hydroxypropyl group, a methoxy group, an acetyl group and a succinyl group.

The DS of acetyl group per number of anhydroglucose unit in HPMCAS is from 0.10 to 2.50, preferably from 0.10 to 1.00, more preferably from 0.25 to 0.65, and even more preferably from 0.40 to 0.55.

The DS of succinyl group per number of anhydroglucose unit in HPMCAS is from 0.05 to 2.50, preferably from 0.10 to 1.00, more preferably from 0.30 to 0.55, and even more preferably from 0.30 to 0.45.

The DS of methoxy group per number of anhydroglucose unit in HPMCAS is not particularly limited, but is preferably from 1.00 to 2.90, more preferably from 1.50 to 2.40.

The MS of hydroxypropoxy group per mole of anhydroglucose unit in HPMCAS is not particularly limited, but is preferably from 0.10 to 1.00, more preferably from 0.10 to 0.40.

Here, the DS of methoxy group, acetyl group, and succinyl group in HPMCAS represents the Degree of Substitution and is the average number of methoxy group, acetyl group, and succinyl group per number of anhydroglucose unit, respectively. The MS of hydroxypropoxy group in HPMCAS represents the Molar Substitution and is the average number of moles of hydroxypropoxy group per mole of anhydroglucose.

It is to be noted that the DS of methoxy group, acetyl group, and succinyl group and the MS of hydroxypropoxy group in HPMCAS can be converted from the values obtained by the method described in "Hypromellose acetate succinate" in each article of the Japanese Pharmacopoeia 18th Edition, respectively.

The method of obtaining HPMCAS is not particularly limited, and it may be produced by any known producing method or obtained as a commercial product.

For example, HPMCAS is produced by esterifying hydroxypropyl methylcellulose with acetylating agent and succinylating agent in the presence of an aliphatic carboxylic acid to obtain a reaction solution, mixing the obtained reaction solution with water to precipitate the same, preparing a suspension containing HPMCAS particles, and subjecting HPMCAS in this suspension to washing, collecting, drying, pulverization, and other processes.

Examples of the neutralizer include ammonia and alkaline substances such as alkylamines including monomethylamine, dimethylamine, and trimethylamine. An alkaline substance with a low boiling point is preferred and ammonia is especially preferred from the viewpoint that it volatilizes in the drying process of the solid preparation producing method described below and remains less residual.

The neutralizer may be used alone or in combination of two or more neutralizers as needed. The neutralizer may be dissolved in water or other solvent as needed and used as a solution.

The degree of neutralization of HPMCAS by a neutralizer is a ratio of the amount of neutralizer actually added to HPMCAS when the amount of neutralizer required to completely neutralize the succinyl group of HPMCAS is defined as 100 mol %, and is calculated as: degree of neutralization = (100 × the amount of neutralizer added / the amount of neutralizer required to completely neutralize the succinyl group of HPMCAS) (unit: mol %). The "degree of neutralization" here is not the percentage that was actually neutralized, but rather an index amount of how much neutralizer was added compared to the equivalent amount that would normally (in theory) be neutralized. Thus, the "degree of neutralization" may exceed 100 mol %.

The degree of neutralization of HPMCAS by the neutralizer is from 130 to 260 mol %, preferably from 140 to 260 mol %, more preferably from 140 to 250 mol %, still more preferably from 150 to 250 mol %, and especially preferably from 200 to 250 mol % of the succinyl group of HPMCAS. When the degree of neutralization is less than 130 mol %, the viscosity reduction of the aqueous composition is insufficient, resulting in poor operability and requiring more time for coating. Spray nozzle blockage may also occur during a coating operation. Further, since the viscosity of aqueous composition is also dependent on the concentration of dissolved HPMCAS, the concentration is sometimes limited due to insufficient viscosity reduction. On the other hand, when the degree of neutralization exceeds 260 mol %, sufficient acid resistance cannot be ensured.

The viscosity of the aqueous composition is not particularly limited as long as it is within the range that can be sprayed for performing coating, but from the viewpoint of operability, the viscosity of the aqueous composition at 20°C is usually preferably 10 to 200 mPa·s, more preferably 40 to 110 mPa·s. Here, the above viscosity can be measured by the viscosity measurement method described in the Japanese Pharmacopoeia 18th Edition.

The aqueous composition of the present invention contains an excess amount of neutralizer from 130 to 260 mol % relative to the succinyl group of HPMCAS, and the above viscosity is lower than that of aqueous composition containing the same amount (i.e., 100 mol %) of neutralizer relative to the succinyl group of HPMCAS. The following is an inferred mechanism by which solution viscosity decreases due to the inclusion of an excessive amount of neutralizer.

It is inferred that due to the presence of an excess amount of neutralizer in the aqueous composition, the cross-linked structure in HPMCAS is decrosslinked and the molecular weight decreases. It is also inferred that the addition of an excess amount of the neutralizer causes electronic repulsion of the neutralizer, which suppresses association and aggregation caused by the carboxylic acid moieties (carboxy group in the succinyl group) of HPMCAS, resulting in a decrease in apparent molecular weight. It is inferred that a decrease in molecular weight or apparent molecular weight will result in a viscosity decrease.

The concentration of HPMCAS in the aqueous composition can be increased or decreased as long as it does not interfere with the coating operation and conditions during solid preparation production, as described below, but from the viewpoint of reduced coating time and ease of operation, it is preferably from 4 to 12% by mass, more preferably from 7 to 10% by mass.

The water content in the aqueous composition is the remainder of HPMCAS and the neutralizer, or is the remainder of HPMCAS, neutralizer, and additives when the aqueous composition contains additives.

An aqueous composition may include an additive(s) as needed. Examples of the additive include, but are not limited to, a lubricant, a colorant, a pigment, a sweetener, and an antifoaming agent.

Examples of the lubricant include talc, magnesium stearate, calcium stearate, colloidal silica, stearic acid, and fumed silica. Talc is preferred from the viewpoint of preventing adhesion between HPMCAS particles during coating.

Examples of the colorant include yellow oxide of iron, red ferric oxide (red), orange essence, brown iron oxide, caramel, light anhydrous silicic acid, food blue No. 5, food yellow No. 4, food yellow No. 4 aluminum lake, food yellow No. 5, food red No. 2, food red No. 3, food red No. 102, food blue No. 2, talc, fluorescein sodium, green tea powder, vitamin C, food lake coloring, carotenoid coloring, flavonoid coloring, and quinone coloring.

Examples of the pigment include rutile titanium dioxide, anatase titanium dioxide, white lead, basic lead sulfate, basic lead silicate, zinc oxide, zinc sulfide, and antimony trioxide.

Examples of the sweetener include monosaccharides and disaccharides such as glucose, fructose, sucrose, lactose, palatinose, trehalose, and xylose; isomerized sugars such as a glucose fructose liquid sugar and a fructose glucose liquid sugar; sugar alcohols such as erythritol, xylitol, lactitol, palatinit, sorbitol and reduced sugar syrup; and high-intensity sweeteners such as sucralose, acesulfame potassium, thaumatin, aspartame; and honey.

Examples of the antifoaming agent include glycerol fatty acid esters, dimethylpolysiloxane, dimethylpolysiloxane-silicon dioxide mixtures, hydrated silica, and silicon dioxide.

The content of the additive may be appropriately selected depending on the kind and the purpose of addition, as long as it does not impair the effects of the invention. For example, from the viewpoint of maintaining the strength and acid resistance of the coating in the solid preparation described below, it is preferably from 0.1 to 50.0 parts by mass relative to 100 parts by mass of HPMCAS.

The aqueous composition may be produced by a method including a step of mixing HPMCAS, water, and a neutralizer to obtain the aqueous composition.

Mixing of HPMCAS and water can be carried out by adding HPMCAS to water or by adding water to HPMCAS, but from the viewpoint of dispersibility of HPMCAS, adding HPMCAS to water is preferred.

The addition of the neutralizer is not particularly limited. For example, water may be appropriately mixed with the neutralizer, followed by adding HPMCAS therein. In another example, HPMCAS may be appropriately mixed with water, followed by adding the neutralizer therein. From the standpoint of dispersibility of HPMCAS, it is preferred that the water may be first mix with the neutralizer.

The mixing of HPMCAS with the water and the neutralizer is not particularly limited. For example, HPMCAS is dispersed in purified water using a propeller mixer or the like, followed by adding the neutralizer to the resulting dispersion liquid to make an aqueous composition. The rotational speed of propeller mixer is preferably from 500 to 1200 rpm from the viewpoint of allowing HPMCAS to sufficiently dissolve.

The temperature for producing the aqueous composition is not particularly limited. From the standpoint of convenience of the production, it is preferably from 5 to 30°C., more preferably from 20 to 30°C.

The mixing time for producing the aqueous composition is preferably from 90 to 210 minutes after adding all of HPMCAS, water and neutralizer, from the viewpoint of sufficiently dissolving HPMCAS in the water

The aqueous composition of the present invention produced in the above manner is suitable as a composition for coating a solid preparation, and is especially suitable as an enteric coating composition, because it is acid-resistant, does not become too viscous, and does not cause blockage of spray nozzle.

2. Solid preparations

Next, described is the solid preparation coated with the aforementioned aqueous composition for enteric coating.

The solid preparation has at least a core containing at least an active ingredient and a coating layer in which the core is coated with the aqueous composition.

The core may be a capsule preparation filled with a filling containing at least the active ingredient.

The active ingredient is not particularly limited as long as it is an orally administrable active ingredient. Examples of the active ingredient include a drug for the central nervous system, a drug for the cardiovascular system, a drug for the respiratory system, a drug for the digestive system, an antibiotic, an antitussive and expectorant, an antihistamine, an antipyretic anti-inflammatory analgesic, a diuretic, an autonomic agent, an antimalarial agent, an antidiarrheal agent, a psychotropic, and vitamins and derivatives thereof.

Examples of the drug for the central nervous system include diazepam, idebenone, paracetamol, naproxen, piroxicam, indomethacin, sulindac, lorazepam, nitrazepam, phenytoin, acetaminophen, ethenzamide, and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as lansoprazole and omeprazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive and expectorant include dihydrocodeine phosphate, noscapine hydrochloride, carbetapentane citrate, isoaminile citrate, and dimemorfan phosphate.

Examples of the antihistamine include chlorpheniramine maleate, diphenhydramine hydrochloride, and promethazine hydrochloride.

Examples of the antipyretic anti-inflammatory analgesic include ibuprofen, diclofenac sodium, flufenamic acid, sulpyrine, aspirin, and ketoprofen.

Examples of the diuretic include caffeine.

Examples of the autonomic agent include dl-methylephedrine hydrochloride, atropine sulfate, acetylcholine chloride, and neostigmine.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, and calcium pantothenate.

Examples of the active ingredient used in a health food include the aforementioned vitamins and derivatives thereof, mineral, carotenoid, an amino acid and amino acid derivatives, a plant extract, and a health food ingredient.

Examples of the mineral include calcium, magnesium, manganese, zinc, iron, copper, selenium, chromium, sulfur, and iodine.

Examples of the carotenoid include beta-carotene, alpha-carotene, lutein, cryptoxanthin, zeaxanthin, lycopene, astaxanthin, and multi-carotene.

Examples of the amino acid include an acidic amino acid, a basic amino acid, a neutral amino acid, and an acidic amino acid amide.

Examples of the acidic amino acid include aspartic acid and glutamic acid.

Examples of the basic amino acid include lysine, arginine, and histidine.

Examples of the neutral amino acid include: a linear aliphatic amino acid such as alanine and glycine; a branched aliphatic amino acid such as valine, leucine, and isoleucine; hydroxy amino acids such as serine and threonine; a sulfur-containing amino acid such as cysteine and methionine; an aromatic amino acid such as phenylalanine and tyrosine; a heterocyclic amino acid such as tryptophan; an imino acid such as proline; and a non-natural amino acid such as tranexamic acid.

Examples of the acidic amino acid amide include asparagine and glutamine.

Examples of the amino acid derivative include acetylglutamine, acetylcysteine, carboxymethylcysteine, acetyl tyrosine, acetylhydroxyproline, 5-hydroxyproline, glutathione, creatine, S-adenosylmethionine, glycylglycine, glycyl-glutamine, dopa, alanyl-glutamine, carnitine, and gamma-aminobutyric acid.

Examples of the plant extract include extracts of Aloe, Propolis, Agaricus subrufescens, Panax ginseng (also known as Korean ginseng), Ginkgo biloba, Curcuma longa (turmeric), curcumin, germinated brown rice, lentinura edodes mycelium (shiitake mushroom mycelium), tian cha, amacha (a Japanese herbal tea made from fermented leaves of Hydrangea macrophylla var. thunbergii), phellinus linteus, Sesamum indicum, Allium sativum, Lepidium meyenii, Ophiocordyceps sinensis, Matricaria chamomilla (commonly known as chamomile), and Capsicum annuum.

Examples of the health food ingredient include royal jelly, dietary fiber, protein, Bifidobacterium, lactic acid bacteria (LAB), chitosan, yeast, glucosamine, lecithin, polyphenol, animal and fish cartilage, pelodiscus sinensis, lactoferrin, cyrenidae, eicosapentaenoic acid (EPA), germanium, enzyme, creatine, carnitine, citric acid, raspberry ketone, coenzyme $Q_{10}$, methylsulfonylmethane, and phospholipid-bound soy peptide.

The amount of active ingredient used can be determined according to the kind of preparation. Two or more kinds of active ingredients may be used together as appropriate. Here, a commercially available active ingredient(s) can be used.

The core containing at least the active ingredient, may include one or more additives selected from an excipient, a binder, a disintegrant, a lubricant (an aggregation inhibitor), a fluidizer, a colorant, and a solubilizing agent for a pharmaceutical compound.

Examples of the excipient include a saccharide such as white soft sugar, lactose, mannitol, and glucose; starch; crystalline cellulose; calcium phosphate; and calcium sulfate.

Examples of the binder include polyvinyl alcohol, polyacrylic acid, polymethacrylic acid, polyvinylpyrrolidone, glucose, white soft sugar, lactose, maltose, dextrin, sorbitol, mannitol, hydroxyethyl cellulose, hydroxypropyl methyl cellulose, hydroxypropyl cellulose, macrogols (e.g., macrogol 4000, macrogol 6000, macrogol 20000), gum arabic, gelatin, agar, and starch (e.g., cornstarch).

Examples of the disintegrant include low-substituted hydroxypropyl cellulose, carmellose or salt thereof, croscarmellose sodium, sodium carboxymethyl starch, cross-polyvinylpyrrolidone, crystalline cellulose, and crystalline cellulose-carmellose sodium.

Examples of the lubricant (the aggregation inhibitor) include talc, magnesium stearate, calcium stearate, colloidal silica, stearic acid, waxes, hardened oil, polyethylene glycol, and sodium benzoate.

Examples of the fluidizer include hydrated silica, light anhydrous silicic acid, and titanium oxide.

Examples of the colorant include yellow ferric oxide, red ferric oxide, food blue No. 1, food blue No. 2, food yellow No. 4, food yellow No. 5, food green No. 3, food red No. 2, food red No. 3, food red No. 102, food red No. 104, food red No. 105, and food red No. 106.

Examples of the solubilizing agent for a pharmaceutical compound include organic acids such as fumaric acid, succinic acid, malic acid, and adipic acid.

The amount of additive used can be appropriately determined depending on the kind of the preparation and other factors. As appropriate, two or more kinds of additives may be used in combination. Further, a commercially available additive(s) may be used.

The shape (dosage form) of the core containing at least the active ingredient is not particularly limited. Examples of the dosage form include tablets, powder, granules, fine granules, pills, troches, and capsules. Tablets are particularly preferred among them.

Examples of the tablet include an uncoated tablet, a sugar-coated tablet, a gelatin-coated tablet, a film-coated tablet (including a multi-layer film-coated tablet), an enteric-coated tablet, and a core-shell tablet (a compressed coated tablet).

Further, the core containing at least the active ingredient may be undercoated with a coating base such as hydroxypropyl methylcellulose, as necessary, before the aqueous composition is coated thereon.

The amount of the aqueous composition coated on the surface of the core containing at least the active ingredient, can be appropriately determined depending on the kind, shape, size, surface condition of the preparation, and the property of the drug and/or additive contained in the preparation. For example, when the shape (dosage form) of the core containing at least the active ingredient is a tablet, the amount of solid content of the aqueous composition is preferably 0.5 to 15 parts by weight, more preferably 5 to 10 parts by weight, based on 100 parts by weight of the core, from the viewpoint of uniformity of the coating amount. Here, the "amount of solid content" of the aqueous composition is the total amount of the components of the aqueous composition other than water and neutralizers, that is, the total amount of HPMCAS, and when the additives are contained as needed, the amount of such additives is also included.

Further, the thickness of the coating layer in tablets is preferably 20 to 30 $\mu$m from the viewpoint of forming a uniform continuous film (layer). The thickness of the coating layer in tablets can be measured using a digital microscope VHX-2000 (Keyence Corporation).

3. Method for producing solid preparation

Next, a method for producing the solid preparation will be described.

The solid preparation is obtained by a producing method that includes the process of applying the aqueous composition to a core containing at least the active ingredient using a coating apparatus or the like, and a subsequent drying process. Examples of the coating apparatus include a pan coating apparatus, a drum-type coating apparatus, a fluidized bed granulation device, and an agitated fluid coating apparatus. Conventionally known coating apparatuses can be used, and examples of the spray device associated with the coating apparatus include an air spray, an airless spray, and a three-fluid spray. In the drying process, known drying methods are used, such as natural drying, drying using a shelf dryer; and a drying method using a ventilation drying type coating apparatus.

[Working examples]

The present invention is specifically described hereunder with reference to working and comparative examples; however, the present invention shall not be limited to them.

Working example 1

To 169.97g of purified water, 20.00 g of HPMCAS having the degree of substitution shown in Table 1 was added at room temperature. Then, an aqueous composition with a HPMCAS concentration of 10.0% by mass was prepared by adding 10.03 g of 10% by mass of an aqueous ammonia solution (an amount to achieve 200 mol % neutralization degree) therein, followed by stirring at 1300 rpm for 180 minutes using a propeller mixer in a water bath at approximately 5°C. The viscosity of the prepared aqueous composition at 20°C was measured using an Ubbelohde type viscometer in accordance with the capillary viscometer method of viscosity measurement in the general test method described in the Japanese Pharmacopoeia18th Edition.

The measured viscosity is listed in Table 1.

Working example 2

An aqueous composition with a HPMCAS concentration of 10.0% by mass was prepared in the same manner as in working example 1, except that the neutralizer was changed to a 40% by mass of an aqueous monomethylamine solution, and the viscosity thereof was measured.

Working example 3

An aqueous composition with a HPMCAS concentration of 10.0% by mass was prepared in the same manner as in working example 1, except that the neutralizer was changed to an approximately 50% by mass of an aqueous dimethylamine solution, and the viscosity thereof was measured.

Working example 4

An aqueous composition with a HPMCAS concentration of 10.0% by mass was prepared in the same manner as in working example 1, except that the neutralizer was changed to a 30% by mass of an aqueous trimethylamine solution, and the viscosity thereof was measured.

Working example 5

An aqueous composition with a HPMCAS concentration of 10.0% by mass was prepared in the same manner as in working example 1, except that the neutralizer was changed to triethylamine, and the viscosity thereof was measured.

Comparative example 1

An aqueous composition with a HPMCAS concentration of 10.0% by mass was prepared in the same manner as in working example 1, except that the degree of neutralization with a 10% by mass of an aqueous ammonia solution was changed to 100 mol %, and the viscosity thereof was measured.

Comparative example 2

An aqueous composition with a HPMCAS concentration of 10.0% by mass was prepared in the same manner as working example 1, except that the neutralizer was changed to a 40% by mass of an aqueous monomethylamine solution and the degree of neutralization was changed to 100 mol %, and the viscosity thereof was measured.

Comparative example 3

An aqueous composition with a HPMCAS concentration of 10.0% by mass was prepared in the same manner as in working example 1, except that the neutralizer was changed to an approximately 50% by mass of an aqueous dimethylamine solution and the degree of neutralization was changed to 100 mol %, and the viscosity thereof was measured.

Comparative example 4

An aqueous composition with a HPMCAS concentration of 10.0% by mass was prepared in the same manner as in working example 1, except that the neutralizer was changed to a 30% by mass of an aqueous trimethylamine solution and the degree of neutralization was changed to 100 mol %, and the viscosity thereof was measured.

Comparative example 5

An aqueous composition with a HPMCAS concentration of 10.0% by mass was prepared in the same manner as in working example 1, except that the neutralizer was changed to triethylamine and the degree of neutralization was changed to 100 mol %, and the viscosity thereof was measured.
Table 1 shows the viscosity measurement results of the aqueous compositions of working examples 1 to 5 and comparative examples 1 to 5. In working examples 1 to 5, the viscosity reduction ratio was calculated from the viscosity values of the corresponding comparative examples in which the degree of neutralization is 100 mol %, and the results are also shown in Table 1.

[Table 1]

| | Degree of substitution or Molar substitution * | | | | Mass concentration of HPMCAS in composition (wt%) | Neutralizer (-) | Degree of neutralization (mol %) | Viscosity (mPa·s) | Viscosity reduction ratio (%) |
|---|---|---|---|---|---|---|---|---|---|
| | MeO Group (-) | HPO Group (-) | Ac Group (-) | Sue Group (-) | | | | | |
| Working example 1 | 1.89 | 0.24 | 0.48 | 0.38 | 10.0 | Ammonia | 200 | 144 | 61 |
| Working example 2 | 1.89 | 0.24 | 0.48 | 0.38 | 10.0 | Monomethylamine | 200 | 114 | 59 |
| Working example 3 | 1.89 | 0.24 | 0.48 | 0.38 | 10.0 | Dimethylamine | 200 | 121 | 59 |
| Working example 4 | 1.89 | 0.24 | 0.48 | 0.38 | 10.0 | Trimethylamine | 200 | 186 | 43 |
| Working example 5 | 1.89 | 0.24 | 0.48 | 0.38 | 10.0 | Triethylamine | 200 | 174 | 46 |
| Comparative example 1 | 1.89 | 0.24 | 0.48 | 0.38 | 10.0 | Ammonia | 100 | 368 | - |
| Comparative example 2 | 1.89 | 0.24 | 0.48 | 0.38 | 10.0 | Monomethylamine | 100 | 278 | - |
| Comparative example 3 | 1.89 | 0.24 | 0.48 | 0.38 | 10.0 | Dimethylamine | 100 | 294 | - |
| Comparative example 4 | 1.89 | 0.24 | 0.48 | 0.38 | 10.0 | Trimethylamine | 100 | 325 | - |
| Comparative example 5 | 1.89 | 0.24 | 0.48 | 0.38 | 10.0 | Triethylamine | 100 | 323 | - |
| * Each value in Meo, Ac, and Suc groups represents the degree of substitution, and each value in HPO group represents the molar substitution. | | | | | | | | | |

EP 4 417 196 A1

Working example 6

To 1259.3 g of purified water, 116 g of HPMCAS having the degree of substitution shown in Table 1 was added at room temperature with stirring using a propeller mixer to disperse the mixture, 39.9 g of 10% by mass of an aqueous ammonia solution (in an amount to achieve the neutralization degree of 140 mol %) was added and stirred for another 150 minutes to prepare an ammonia-neutralized aqueous solution of HPMCAS. Furthermore, 34.8 g (30 parts by weight with respect to 100 parts by weight of HPMCAS) of talc (crown talc, manufactured by Matsumura Sangyo Co., Ltd.) was added to the aforementioned ammonia-neutralized aqueous solution thus obtained and stirred for 30 minutes to prepare an aqueous composition with a HPMCAS concentration of 8.0% by mass, and the viscosity was measured in the same manner as above.

Under the following conditions, the prepared aqueous composition was used to coat a placebo tablet (manufactured by AKIYAMA JOZAI CO. LTD) with a diameter of 8 mm, a radius of curvature of 6.5 mm, and a tablet weight of 190 mg until solid content weight reached to 9 parts by mass (112.5 parts by mass as aqueous composition) relative to 100 parts by mass of the placebo tablet. After completion of the coating, drying and polishing were performed simultaneously in the same coating apparatus at an air supply temperature of 60°C and a rotation speed of the pan of 18 rpm for 30 minutes to obtain solid preparations (enteric-coated tablets).

> Device: Ventilation type pan coater (inner diameter 33 cm)
> Charged amount: 1 kg
> Air supply temperature: 80°C
> Exhaust gas temperature: from 44 to 46°C
> Air supply flow rate: 1 m³/min
> Rotation speed of a pan: 18 rpm
> Spray rate: from 6.0 to 6.4 g/min
> Spray air pressure: 200 kPa

The disintegration test of 50 enteric-coated tablets thus obtained was conducted in accordance with the Japanese Pharmacopoeia by using 900 ml of the 1st fluid (pH 1.2) for disintegration test as described in the Japanese Pharmacopoeia 18th Edition. After 2 hours from the disintegration test, the number of tablets whose enteric coating had ruptured or broken (defective tablets) was measured and the tablet defect ratio was calculated to evaluate acid resistance.

$$\text{Tablet defect ratio } (\%) = \text{Number of defective tablets} / 50 \times 100$$

Further, the disintegration test of 6 enteric-coated tablets thus obtained was conducted by using 900 ml of the 2nd fluid (pH 6.8) for disintegration test as described in the Japanese Pharmacopoeia 18th Edition, and the disintegration time was measured.

Additionally, 200 mL of a 0.4% by mass aqueous sodium hydroxide solution was added to the 5 enteric-coated tablets obtained at room temperature and stirred with a magnetic stirrer for 180 minutes to prepare an aqueous solution for test. The amount of ammonia contained in the prepared aqueous solution for the test was measured with a Quick Ammonia Meter Model AT-2000 (manufactured by Central Kagaku Corp.), and the amount of residual ammonia in the enteric-coated tablets was calculated.

The measured tablet defect ratio, the disintegration time and the amount of residual ammonia are shown in Table 2.

In the evaluation of acid resistance, a tablet defect ratio of 0% was defined as having sufficient acid resistance.

The aforementioned Quick Ammonia Meter Model AT-2000 is a measuring device that is capable of quickly and easily measuring ammonia nitrogen by the coulometric titration method.

Working example 7

The aqueous composition was prepared in the same manner as in working example 6, except that the concentration of HPMCAS was changed to 9.0% by mass and the degree of neutralization was changed to 200 mol %. Coated tablets were produced by using the prepared aqueous composition, and various properties of the coated tablets were evaluated as in working example 6.

Working example 8

The aqueous composition was prepared in the same manner as in working example 6, except that the concentration of HPMCAS was changed to 9.5% by mass and the degree of neutralization was changed to 250 mol %. Coated tablets were produced by using the prepared aqueous composition, and various properties of the coated tablets were evaluated as in working example 6.

Working example 9

The aqueous composition was prepared in the same manner as in working example 6, except that the concentration of HPMCAS was changed to 10.0% by mass and the degree of neutralization was changed to 250 mol %. Coated tablets were produced by using the prepared aqueous composition, and various properties of the coated tablets were evaluated as in working example 6.

Comparative example 6

The aqueous composition was prepared in the same manner as in working example 6, except that the concentration of HPMCAS was changed to 9.0% by mass and the degree of neutralization was changed to 100 mol %. Attempts were made to produce coated tablets by using the prepared aqueous composition, but blockage of the spray nozzle occurred during the coating process.

Comparative example 7

The aqueous composition was prepared in the same manner as in working example 6, except that the concentration of HPMCAS was changed to 9.5% by mass and the degree of neutralization was changed to 270 mol %. Coated tablets were produced by using the prepared aqueous composition, and various properties of the coated tablets were evaluated in the same manner as in working example 6.

Comparative example 8

The aqueous composition was prepared in the same manner as in working example 6, except that the concentration of HPMCAS was changed to 10.0% by mass and the degree of neutralization was changed to 300 mol % in the preparation of the aqueous composition in working example 6. Coated tablets were produced by using the prepared aqueous composition, and various properties of the coated tablets were evaluated in the same manner as in working example 6.

The results of the working examples show that when the degree of neutralization of HPMCAS by the neutralizer is set within the range from 130 mol % to 260 mol %, no blockage of the spray nozzle occurs and a good enteric-coated tablet with a 0% defect ratio of coated tablets is obtained. Further, from working examples 8 and 9, it was found that the spraying time of the aqueous composition (coating liquid) can be reduced by increasing the mass concentration of HPMCAS in the aqueous composition, even if the degree of neutralization of HPMCAS by the neutralizer is the same.

From the results of the working and comparative examples, it was found that the defect ratio of coated tablets does not reach 0% when the degree of neutralization of HPMCAS by the neutralizer is greater than 260 mol %. Moreover, from working example 7 and comparative example 6, it was found that even if the HPMCAS mass concentration is the same, increasing the degree of neutralization decreases the viscosity of the composition and prevents spray nozzle blockage.

[Table 2]

| | Producing method | | | | | Enteric-coated tablet | | |
|---|---|---|---|---|---|---|---|---|
| | Mass concentration of HPMCAS in composition (wt%) | Degree of neutralization (mol %) | Viscosity (mPa·s) | Spraying time (min) | Blockage of spray nozzle (-) | Tablet defect ratio (%) | Disintegration time (min.) | Amount of residual ammonia (mg/T) |
| Working example 6 | 8.0 | 140 | 102 | 181 | Not blocked | 0 | 7.9 | 0.35 |
| Working example 7 | 9.0 | 200 | 104 | 163 | Not blocked | 0 | 7.2 | 0.31 |
| Working example 8 | 9.5 | 250 | 119 | 158 | Not blocked | 0 | 6.3 | 0.27 |
| Working example 9 | 10.0 | 250 | 138 | 146 | Not blocked | 0 | 6.3 | 0.39 |
| Comparative example 6 | 9.0 | 100 | 255 | - | Blocked | - | - | - |
| Comparative example 7 | 9.5 | 270 | 118 | 152 | Not blocked | 2 | 5.9 | 0.35 |
| Comparative example 8 | 10.0 | 300 | 134 | 153 | Not blocked | 82 | 5.4 | 0.30 |

**Claims**

1.  An aqueous composition comprising: hydroxypropyl methyl cellulose acetate succinate having an acetyl group substitution degree from 0.10 to 2.50 and a succinyl group substitution degree from 0.05 to 2.50 per number of anhydroglucose unit; a neutralizer; and water, wherein the degree of neutralization of the hydroxypropyl methyl cellulose acetate succinate by the neutralizer is from 130 to 260 mol %.

2.  The aqueous composition according to claim 1, wherein the neutralizer is at least one selected from the group consisting of ammonia and alkylamine.

3.  The aqueous composition according to claim 1 or 2, wherein the aqueous composition is for enteric coating.

4.  A method for producing a solid preparation comprising: applying the aqueous composition according to claim 3 to a core containing at least an active ingredient; and drying the same thereafter.

**EUROPEAN SEARCH REPORT**

Application Number

EP 24 15 7768

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DU YING ET AL: "Effect of neutralization on physicochemical/mechanical properties of hydroxypropylmethylcellulose acetate succinate", MATERIALS CHEMISTRY AND PHYSICS, ELSEVIER SA, SWITZERLAND, TAIWAN, REPUBLIC OF CHINA, vol. 249, 122963, 4 April 2020 (2020-04-04), XP086190074, ISSN: 0254-0584, DOI: 10.1016/J.MATCHEMPHYS.2020.122963 [retrieved on 2020-04-04] * pages 1-2; table 1 * * page 7, paragraph 4. * ----- | 1-3 | INV. A61K9/28 |
| X | EP 3 718 538 A1 (SHINETSU CHEMICAL CO [JP]) 7 October 2020 (2020-10-07) * page 2; table 1 * * page 3, paragraph 0018-0019 * * page 4, paragraph 0025 * * page 6, paragraph 0057-0060 * * examples 6-8, 18-20 * ----- | 1-4 | |

TECHNICAL FIELDS SEARCHED (IPC)

A61K

The present search report has been drawn up for all claims

| Place of search | Date of completion of the search | Examiner |
|---|---|---|
| The Hague | 27 June 2024 | van de Wetering, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 7768

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2024

| Patent document cited in search report | | Publication date | Patent family member(s) | | Publication date |
|---|---|---|---|---|---|
| EP 3718538 | A1 | 07-10-2020 | CN | 111789960 A | 20-10-2020 |
| | | | EP | 3718538 A1 | 07-10-2020 |
| | | | JP | 7050714 B2 | 08-04-2022 |
| | | | JP | 2020169142 A | 15-10-2020 |
| | | | KR | 20200117883 A | 14-10-2020 |
| | | | US | 2020315977 A1 | 08-10-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 55098120 A
- JP 2018532013 T

**Non-patent literature cited in the description**

- Japanese Pharmacopoeia